(19) **Europäisches Patentamt European Patent Office Office européen des brevets**

(11) **EP 2 845 002 B1**

(12) **EUROPEAN PATENT SPECIFICATION**

(45) Date of publication and mention of the grant of the patent:
**13.07.2016 Bulletin 2016/28**

(21) Application number: **13735394.2**

(22) Date of filing: **03.05.2013**

(51) Int Cl.:
***G01N 33/49*** *(2006.01)*

(86) International application number:
**PCT/IB2013/000826**

(87) International publication number:
**WO 2013/164676 (07.11.2013 Gazette 2013/45)**

(54) **METHOD TO ANALYZE THE CLUSTER FORMATION PROCESS IN A BIOLOGICAL FLUID AND CORRESPONDING ANALYSIS APPARATUS**

VERFAHREN ZUR ANALYSE EINES CLUSTERBILDUNGSVERFAHRENS IN EINER BIOLOGISCHEN FLÜSSIGKEIT UND ENTSPRECHENDE ANALYSEVORRICHTUNG

PROCÉDÉ POUR ANALYSER LE PROCESSUS DE FORMATION D'AMAS DANS UN FLUIDE BIOLOGIQUE ET APPAREIL D'ANALYSE CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.05.2012 IT UD20120079**

(43) Date of publication of application:
**11.03.2015 Bulletin 2015/11**

(73) Proprietors:
• **Universita' Degli Studi di Udine 33100 Udine (IT)**
• **Centro Di Riferimento Oncologico 33081 Aviano (IT)**

(72) Inventors:
• **AFFANNI, Antonio I-26041 Casalmaggiore (IT)**
• **COZZI, Maria, Rita I-33085 Maniago (IT)**
• **DE MARCO, Luigino I-33085 Maniago (IT)**

• **MAZZUCATO, Mario I-30023 Concordia Sagittaria (IT)**
• **SPECOGNA, Ruben I-33010 Pagnacco (IT)**
• **TREVISAN, Francesco I-33100 Udine (IT)**

(74) Representative: **Petraz, Gilberto Luigi et al GLP S.r.l. Viale Europa Unita, 171 33100 Udine (IT)**

(56) References cited:
**WO-A1-2007/068727     US-A1- 2006 211 071 US-B2- 7 005 857**

• **HIRONO T ET AL: "Microfluidic image cytometry for measuring number and sizes of biological cells flowing through a microchannel using the micro-PIV technique; Microfluidic image cytometry", MEASUREMENT SCIENCE AND TECHNOLOGY, IOP, BRISTOL, GB, vol. 19, no. 2, 1 February 2008 (2008-02-01), page 25401, XP020129404, ISSN: 0957-0233**

## Description

FIELD OF THE INVENTION

The present invention concerns a method to analyze the cluster formation process, such as platelet clusters or red corpuscle clusters or other, in a biological fluid such as blood or other hematic fluids, whether animal or human, and mixtures of said fluid with additive substances.

In particular, the present invention provides to study "in vitro" the phenomenon of growth over time of the clusters in conditions of controlled flow of the fluid, in order to evaluate for example the onset of cardiovascular pathologies, such as potential risks of thrombosis in "silent" subjects, and potential risks of hemorrhages in subjects with disorders of the hemostatis, both congenital and acquired. Furthermore, the method according to the present invention allows to monitor the effectiveness of anti-thrombotic or other drugs.

The present invention also concerns an apparatus to analyze the clustering process in the biological fluid.

BACKGROUND OF THE INVENTION

The coagulation process is the physiological process which stops blood loss from damaged blood vessels, and is essential to guarantee a person's safety in the event of hemorrhages.

Thrombosis is an undesired formation of a hemostatic blockage or thrombus inside a blood vessel or the cardiovascular system.

As is known, the adhesive and clustering capacity of the platelets and the formation of fibrin normally control repairs to tissue damage. The activation of the platelets leads to the formation of a mass of clustered platelets that form a stopper to stop hemorrhages.

Sometimes the platelets can exasperate the repair process, since they are activated improperly if there is a pathological change in the hemostatic process, for example arteriosclerosis which can give rise to a dramatic event which leads to the occlusion of the blood vessel: thrombosis.

A pathological hemostatic event, whether it is thrombotic-ischemic, due to a sudden lack of blood flow in a blood vessel, as in the case of the coronaries in myocardial infarction, or hemorrhagic, is a severe problem for the cardiovascular system. These problems derive from cardiovascular alterations that have been acquired over time, such as arteriosclerosis, aneurisms, atero-venous shunts, vasculitis, anomalies in the cardiac valves, atrial fibrillations and conduction diseases, venous and arterial thromboses or other.

Furthermore, these pathological occurrences are difficult to diagnose since they are also present in subjects who, in their normal social life, have never experienced any pathological events and who, for example, on the occasion of surgical operations, may incur serious hemorrhagic or thrombotic-ischemic events.

For this reason it is fundamentally important, in the clinical field, to use a functional "ex vivo" test that will allow to identify potential thrombotic and hemorrhagic risks in "silent" subjects for cardiovascular pathological events.

To this end the European patent EP2010903 is known, which concerns an apparatus for the diagnosis, prognosis and pharmacological monitoring of the thrombotic-ischemic and hemorrhagic pathology affecting the cardiovascular system.

In particular, this known apparatus comprises a chamber, also called perfusion chamber, for the passage or flow of hematic liquids.

The perfusion chamber is provided with micro-channels in which, using pumping and suction means, the blood is made to flow, said blood having been previously taken from the patient in a test tube, and suitably treated with anticoagulants such as for example heparin, citrate or suchlike, and with fluorescent probes that function as optical markers, such as for example quinacrine, phycoerythrin or suchlike.

A related apparatus is known from US 2006/0211071.

Optical acquisition means, coordinated with the markers for fluorescence analysis, acquire images relating to the development of the hemostatic processes that lead to the formation of thrombi inside the micro-channels.

A processing unit processes the images acquired and supplies indications concerning the behavior of the patient's hemostasis.

The known optical acquisition means can be for example confocal optical microscopes, or inverted optical microscopes.

Using an inverted microscope, the formation of the thrombus is quantified through a two-dimensional digital image. Using this approach the three-dimensional reconstruction of the formation of the thrombus can only be extrapolated indirectly from the two-dimensional one, and may not reflect its real size.

This problem can be solved using the confocal microscope, and on this point see the publications by M. Mazzucato, M. R. Cozzi, P. Pradella, D. Perissinotto, A. Malmstrom, M. Morgelin, P. Spessotto, A. Colombatti, L. De Marco, R. Perris, Vascular, "PG-M/versican variants promote platelet adhesion at low shear rates and cooperate with collagens to induce aggregation" FASEB J. 16 (2002), 1903-1916; by M. Mazzucato, P. Pradella, M.R. Cozzi, L. De Marco, Z. M. Ruggeri, "Sequential cytoplasmic calcium signals in a 2-stage platelet activation process induced by the glycoprotein Ibalpha mechanoreceptor", Blood 100 (2002) 2793-2800; by M. Mazzucato, M. R. Cozzi, P. Pradella, Z. M. Ruggeri, L. De Marco, "Distinct roles of ADP receptors in von Willebrand factormediated platelet signaling and activation under high flow ", Blood 104 (2004), 3221-3227; by A. Casonato, L. De Marco, L. Gallinaro, M. Sztukowska, M. Mazzuccato, M. Battiston, A. Pagnan, Z.M. Ruggeri, "Altered von Willebrand factor subunit proteolysis and multimer processing associated with the Cys2362Phe mutation in the B2 domain ", Thrombosis and Haemostasis 97 (2007) 527-533; by M. Mazzucato, M. R. Cozzi, M. Battiston, M. Jandrot-Perrus, M. Mongiat, P. Marchese, T. J. Kunicki, Z. M. Ruggeri, L. De Marco, "Distinct spatio-temporal Ca2+ signaling elicited by integrin alpha2β1 and glycoprotein V1 under flow", Blood 114 (2009), 2793-280.

However, the sizes of a confocal microscope, and above all its cost, limit its use to sophisticated research laboratories.

Furthermore, since this is a real-time reconstruction, the monochrome laser source used to illuminate the platelet clusters during the hemostasis process interacts greatly with the process itself, transferring energy and thus altering or compromising the dynamics thereof. Simpler and cheaper devices are also widespread, but are less accurate in monitoring cells in flow conditions that integrate fluidic channels located directly on lensless CMOS chips, also functioning in conditions of fluorescence with fields of vision greater than cm^2 and a resolution of a few microns, see for example U. Gurkan, S. Moon, H. Gecki, F. Xu, S. Wang, T. J. Lu and U. Demirci, "Miniaturized lensless imaging systems for cell and microorganism visualization in point-of-care testing ", Biotechnol. J. 2011, 6, pp. 138-149.

Methods are also known for analyzing platelet clustering in whole blood by measuring the electrical resistance between two electrodes immersed in the whole blood, for example as described in D. C. Cardinal, R. J. Flower, "The electronic aggregometer: A novel device for assessing platelet behavior in blood", J. Pharmacol. Methods, 1980, 3, 135-158.

Methods are also known for the in vivo analysis of blood stasis phenomena (see T. Dai, A. Adler, "In vivo blood characterization from bioimpedance spectroscopy of blood pooling", IEEE Trans. Instr. Meas., 2009, 58, 3831-3838) and for monitoring the viscosity of blood during cardiosurgical operations using impedance spectroscopy techniques (see G.A.M. Pop, T.L.M. de Backer, M. de Jong, P.C. Struijk, L. Moraru, Z. Chang, H. G. Goovaerts, C. J. Slager, A. J. J. C. Bogers, "On-line electrical impedance measurement for monitoring blood viscosity during on-pump heart surgery", Eur. Surgical Res., 2004, 36, 259-265).

The Cole-Cole parameters of spectral induced polarization detected using impedance spectroscopy can be correlated to the hematocrit (see Y. Ulgen, M. Sezdi, "Physiological quality assessment of stored whole blood by means of electric measurements", Med. Bio. Eng. Comput., 2007, 45, 653-660), with the quality of the blood (see Y. Ulgen, M. Sezdi, "Hematocrit dependence of the Cole-Cole parameters of human blood", IEEE Proc. 2nd Int. Biomed. Eng. Days, 1998, 71 - 74), and with viscosity.

In particular, an (exponential) logarithmic increase is observed in the capacity associated with the cell membrane as the fibrinogen increases (respectively of the hematocrit). Because of the insulating membrane of the red blood cells, which is non-conductive below 200kHz, the electrical resistance of the whole blood at low frequencies greatly depends on the hematocrit, as described in T. Yamagata, H. Fujisaki, "Investigation of electrical resistivity changes of human blood during dynamic exercise", Trans. Electrical and Electronic Engineering, IEEJ 2008, 3, 79-83. Above 10 MHz, the membrane behaves like a short circuit and the conductance measured is a weighted mean of the conductivities associated with the plasma and the intracellular fluid.

A quantitative model of blood conductivity in conditions of stationary laminar flow inside cylindrical rigid tubes is described in A. E. Hoetink, T. J. C. Faes, K. R. Visser, R. M. Heethaar, "On the flow dependency of the electrical conductivity of blood", IEEE Trans. Biomed. Eng. 2004, 51, 1251-1261, that is, a model based on an extension of the Maxwell-Fricke theory which takes into account the effect of the orientation and deformation of the ellipsoid particles that are induced by shear stress. An extension of this analysis to also take into account a pulsatile flow is described in R. L. Gaw, B. H. Cornish, B. J. Thomas, "The Electrical Impedance of Pulsatile Blood Flowing Through Rigid Tubes: A Theoretical Investigation", IEEE Trans. Biomed. Eng. 2008, 55, 721-727.

In K. Asami, K. Sekine, "Dielectric modelling of erythrocyte aggregation in blood", J. Phys. D: Appl. Phys. 2007, 40, 21972204, the dielectric behavior of whole blood is simulated using a model of the erythrocytes which consists of a disc covered by a membrane, and the model of an aggregation of erythrocytes consists of a pile of discs with a regular spacing.

Furthermore, in O. Baskurt, M. Uyuklu, S. Ozdem and H. J. Meiselman, "Measurement of red blood cell aggregation in disposable capillary tubes", Clinical Hemorheology and Microcirculation 47, pp. 295-305, 2011, and in O. Baskurt, M. Uyuklu and H. J. Meiselman, "Simultaneous monitoring of electrical conductance and light transmittance during red blood cell aggregation", Biorheology 46, pp. 239-249, 2009, techniques were experimented measuring and correlating electrical conductance (by means of 2 electrodes positioned at start and end of the channel) and transmittance of the transverse light with respect to the fluidic channel where the erythrocyte aggregation occurs per shear rate of 500 1/s per 75 sec.

In O. Kwon, J. K. Seo, E. J. Woo and J-R. Yoon, "Electrical Impedance Imaging for Searching anomalies", Comm. Korean Math. Soc. 16 (2001), No. 3, pp. 459-485, the reconstruction is evaluated, from the view point of inverse problems, in a two-dimensional disc, of the geometry (position and sizes) of a limited number of 2D defects, with a different conductivity from that of the disc, starting from a measurement of potentials in a high number of points (>30) distributed uniformly along the whole edge of the domain. At the same points a required distribution of current is also applied, using boundary-Neumann conditions (that is, on the derivative normal with respect to the edge of the potential) so as to guarantee zero sum and uniform density in the disc. The potentials are also normalized so as to guarantee zero mean.

In recent times considerable efforts have been made and increasing attention has been paid to designing microfluidic devices with different geometries, for example that can be personalized upon request from the user, both channels with minimum sizes of a few microns and also electrodes with minimum sizes of some tens of microns, as blood cell counters (see N. Piacentini, D. Demarchi, P. Civera, M. Knaflitz, "Blood Cell Counting by Means of Impedance Measurements in a Microsystem Device", Proc. 30th Int. IEEE EMBS Conf. 2008, 4824-4827) or cell separators (see Y. Nakashima, S. Hata, T. Yasuda, "Blood plasma separation and extraction from a minute amount of blood using dielectrophoretic and capillary forces", Sens. Actuators B 2010, 145, 561-569), so that various suppliers have developed the microtechnologies that allow these to be made.

These technologies for making the channels and the electrodes integrated therein easily allow viewing with the optical or conventional fluorescence microscope, inverted or confocal, and to measure the electrical behavior of an impedance assessment type, using instruments for measuring impedance of the commercial type (LCR Meter Agilent HP4284A with class <0.5%) or developed ad hoc.

An automatic method is also known for monitoring cell behavior, allowing to observe for example cell proliferation, cell adhesion and the spread of the adhesion, the mobility, poration of cell membranes. This method consists of a set of electrodes printed on a gold film, fed by voltages, alternate currents with variable frequency and amplitude; between pairs of electrodes (circular and with a diameter of 250 microns) both the module Z of impedance is measured, and in other methods the module and phase of the impedance. Given that cell membranes at the frequencies of use (<200 kHz) are practically insulating, morphological changes in the cell disposition modify the paths of the electric currents, causing a consequent variation in the impedance measured at the pairs of electrodes.

Different matrices of electrodes exist, each with a diameter of 250 microns, both for stationary cell cultures and also in perfusion conditions, even with a high shear rate with a channel that is 50 mm long, 5 mm wide and 0.4 mm high.

Complete kits are also known, with a total of 10 fluidic and electrical exits, able to be personalized and studied for microscopy, both traditional and fluorescence, with closed channels of variable length, variable heights from tens of microns and widths in the order of 100 microns.

Solutions are also known in which optical fibers are associated with the microfluidic channel in which the blood passes, for the purposes of illumination and to receive the light emitted focused.

With regard to the detection of electrical quantities for analyzing processes concerning the blood, the following documents are also known: document KR2008015212, in which two electrodes are used in contact with the blood, stationary in a cylindrical container, and the impedance is measured and acquired; document SU1278697, in which the formation of spatial coagulation structures of the blood is identified, by comparing conductivity measurements in continuity and in frequency, and the prevalence of conductivity in continuity compared to conductivity in frequency indicates the event; and document US-B2-6797150 in which the concentration of glucose, the hematocrit etc. of a volume of biological sample, for example blood, is evaluated with measurements of capacity and resistance in the domain of time and in frequency. The biological sample occupies the whole volume between two electrodes of an electrochemical cell characterized by a pair of flat electrodes, facing each other at a distance 1; S indicates the conductive area of contact with the electrodes of the cell sample, the capacity C depends on S (in the hypothesis that the serial capacity of the double layer of ions at the interface between electrodes and cells is constant), the resistance R depends on 1/S, their ratio C/R is proportional to the square of S. From this ratio the area covered by the sample and consequently the volume S 1 is deduced.

Document US-A1-20080297169 provides to measure a fraction of particles in biological fluids (for example hematocrit in the blood, coagulation speed, prothrombin time PT, activated partial thromboplastin time APPT, activated clotting time ACT, thrombin clotting time (TCT) and non-biological fluids. It also allows to determine the concentration of an analyte in a sample of blood, plasma, serum, urine etc. The fluids are stationary. The device contains the necessary reagents. The technique is based on measuring impedance in AC or resistance in DC between pairs of electrodes. A standard thermostatic system keeps the temperature for example at 35°C. The electrical feeds are standard, as is the system for acquiring the electrical voltage and current signals for calculating resistance or impedance.

Document WO-A1-2005114140 describes a device for measuring the coagulation of whole blood or the prothrombin time in the serum. It studies the motion of a particle of ferromagnetic material driven by magnetic forces inside the chamber containing the sample of biological fluid to be examined.

Finally, document WO-A1-2011073481 describes a multi-electrode system which measures the change in impedance due to the presence of a particular component in a solution of different nature (biological or not). The change in impedance is connected to the particular component present.

The documents cited above relating to impedance measurements are not able to determine with reasonable certainty the evolution of growth of a platelet cluster in conditions of flow of the hemostatic fluid, but only in stationary conditions of the hemostatic fluid, for example in a test tube.

This analysis of the hemostatic process could be obtained with optical techniques but the apparatuses required are particularly complex to manage and use, and are particularly costly and do not justify their application in outpatient structures.

One purpose of the present invention is to perfect a method to analyze the cluster formation process in a biological fluid, such as blood, in flow conditions, in order to estimate the development over time of the growth of the cluster, that is, of its volume.

Another purpose of the present invention is to perfect a method of analysis that is reliable and precise and that allows to acquire substantially in real time and analyze the clustering process in its entirety and complexity, with a high degree of reliability and precision.

Another purpose of the present invention is to obtain an apparatus for analyzing the clustering process in a biological fluid which is economical, easily transportable and suitable to be used also in outpatient structures.

Another purpose of the present invention is to obtain an apparatus that is reliable, precise, and allows to monitor, to acquire in real time and to analyze the formation of clusters in dynamic mode.

The Applicant has devised, tested and embodied the present invention to overcome the shortcomings of the state of the art and to obtain these and other purposes and advantages.

SUMMARY OF THE INVENTION

The present invention is set forth and characterized in the independent claims, while the dependent claims describe other characteristics of the invention or variants to the main inventive idea.

In accordance with the above purposes, a method to analyze the formation process of clusters, such as clusters of platelets or red corpuscles or suchlike, in a biological fluid such as blood, hematic fluids or other fluids, is used for the "in vitro" study of the growth phenomenon over time of the clusters.

The method comprises at least a step in which the biological fluid is made to flow through at least one micro-channel of a perfusion chamber, and an optical acquisition step, using an optical acquisition device, during which, in at least one investigation area of the micro-channel, images are acquired relating to the formation of clusters. There is a reactive substrate present in the micro-channel, such as a cyto-adhesive substrate, to stimulate the clustering process in the biological fluid.

According to one feature of the present invention, the method also comprises an impedance assessment step, associated to the optical acquisition step, during which, by means of impedance assessment means disposed in correspondence to the investigation area of the micro-channel, a measurement is carried out over time of the impedance of the biological fluid. The impedance detected is correlated to the formation of clusters.

The method also comprises a processing step, using a processing unit, during which the volume of the clusters which form in the investigation area is determined, correlating, over time, information relating to the images acquired in the optical acquisition step with impedance data of the biological fluid acquired in the impedance assessment step.

In this way, by integrating the optical measurements and the impedance measurements relating to the clusters that are gradually formed during the clustering process, it is possible to obtain an indication of the volume of development of the clusters so as to evaluate, for example, the onset of cardiovascular pathologies, or to study the effect of particular drugs. The measurement of the volume is obtained without knowing in advance where the cluster or thrombus will form with respect to the disposition of the impedance assessment means.

According to another feature of the present invention, during the processing step, from the images acquired, a base area is estimated of the clusters that are formed in the investigation area.

According to another feature of the present invention, the base area of the clusters is determined as a function of the light intensity measured from the image.

In some forms of embodiment of the present invention, in the processing step a shape function is determined which is directly proportional to the light intensity measured from the image.

According to another form of embodiment, it is advantageous to provide that the shape function is processed, as a function of said base area of the clusters, so as to approximate it to the development of the impedance measurements acquired in said impedance assessment step, and to determine the volume of said cluster. In other words, a shape function is identified which reproduces better the impedance measurements that have been acquired.

In other forms of embodiment, during the processing step, the impedance data are correlated to an average height of the clusters which form in the investigation area.

The present invention also concerns the apparatus for analyzing the clustering process that implements a method as described above.

Some forms of embodiment of the present invention provide that the impedance assessment means comprise electrodes fed by electrical feed devices and to which measurement devices are associated in order to detect the impedance data.

It may also be provided that from pairs of the electrodes the electrical voltages are detected, and the current passing through is detected for the subsequent evaluation of the impedance. In one form of embodiment of the present invention, the impedance assessment means comprise at least a first electrode and a second electrode, both having an oblong development and disposed parallel to each other, transverse to the longitudinal development of the micro-channel. The investigation area is comprised between the first electrode and the second electrode and is sized, merely by way of example, in the order of micrometers.

In other forms of embodiment it may be provided to use two or more pairs of electrodes, used to carry out distinct measurements of voltage and current.

According to a variant, the impedance assessment means comprise a plurality of elementary measuring cells angularly offset with respect to each other and each provided with at least a pair of said electrodes.

In this way it is possible to provide that each of the elementary measuring cells detects an impedance assessment value of a portion of the investigation area. Subsequently, to determine the volume of the cluster, a weighted mean of the information detected is performed.

According to one form of embodiment, each of the elementary measuring cells comprises a first, central electrode, surrounded by a second electrode, substantially U-shaped.

BRIEF DESCRIPTION OF THE DRAWINGS

These and other characteristics of the present invention will become apparent from the following description of one form of embodiment, given as a non-restrictive example with reference to the attached drawings wherein:

- fig. 1 is a schematic representation of an apparatus for analyzing the formation process of clusters according to the present invention;
- fig. 2 is a plan view of a detail of fig. 1;
- fig. 3 is a perspective view of a component of the detail in fig. 2;
- fig. 4 is a view of a detail of fig. 2;
- fig. 5 is a variant of fig. 4;
- fig. 6 is an image of the clustering phenomenon;
- fig. 7 is a view of a shape function obtained by processing the image in fig. 6;
- fig. 8 is a graph showing the development of the impedance module as a function of the frequency of feed of the blood without clustering, and with clustering;
- fig. 9 is a graph showing the development over time of the impedance module of the blood with or without induced clustering, for a frequency of feed of 150kHz;
- figs. 10, 10a-10d, 11, 11a-11d, 12, 12a-12d, 13 and 13a-13c are representations of the induced clustering phenomenon detected during the induced clustering process.

To facilitate comprehension, the same reference numbers have been used, where possible, to identify identical common elements in the drawings. It is understood that elements and characteristics of one form of embodiment can conveniently be incorporated into other forms of embodiment without further clarifications.

DETAILED DESCRIPTION OF SOME FORMS OF EMBODIMENT

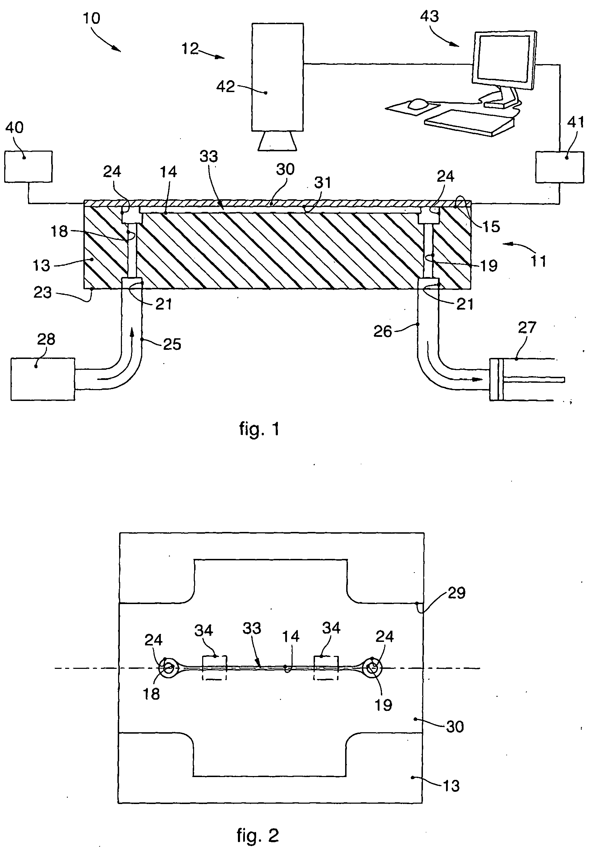

With reference to fig. 1, an apparatus for analyzing the formation process of clusters in a biological fluid according to the present invention is indicated in its entirety by the reference number 10, and comprises at least a perfusion chamber 11 in which a biological fluid is made to flow, in this case blood, and an optical acquisition device 12 suitable to acquire images concerning the flow conditions of the blood in the perfusion chamber 11.

Hereafter in the description we shall refer to the specific case of the formation of platelet clusters, although it is quite evident that a similar description can be given for other clusters such as clusters of red corpuscles or suchlike.

The fluid to be analyzed can be biological fluid, blood, hematic fluids, both animal or human, and mixtures of the fluid with additive substances.

The perfusion chamber 11 (figs. 1, 2 and 3) comprises a base body 13 provided with at least one microgroove 14 made on a first surface 15 of the base body 13, having a mainly longitudinal development and a rectangular section, for example 0.4 mm wide and 0.2 mm high.

In correspondence with each of the terminal ends of the microgroove 14, and orthogonally to the thickness of the base body 13, an inlet channel 18 and respectively an exit channel 19 are made for the blood that is made to flow through the microgroove 14.

The inlet channel 18 and exit channel 19 have a connection end 21 (fig. 1) made in correspondence with a second surface 23 of the base body 13, which is opposite the first surface 15, and a flared end 24 that opens toward the microgroove 14.

In correspondence with the connection end 21 of the inlet channel 18 an inlet pipe 25 for the blood is connected, while in correspondence with the exit channel 19 an exit pipe 26 for the blood is connected.

An element 28 for containing the blood is associated with the inlet pipe 25, while a pumping device is associated with the exit pipe 26, in this case a syringe pump 27, which aspirates the blood from the inlet pipe, making it pass through the microgroove 14 at a constant flow rate and a speed gradient, or shear rate, not less than 3000 s^-1.

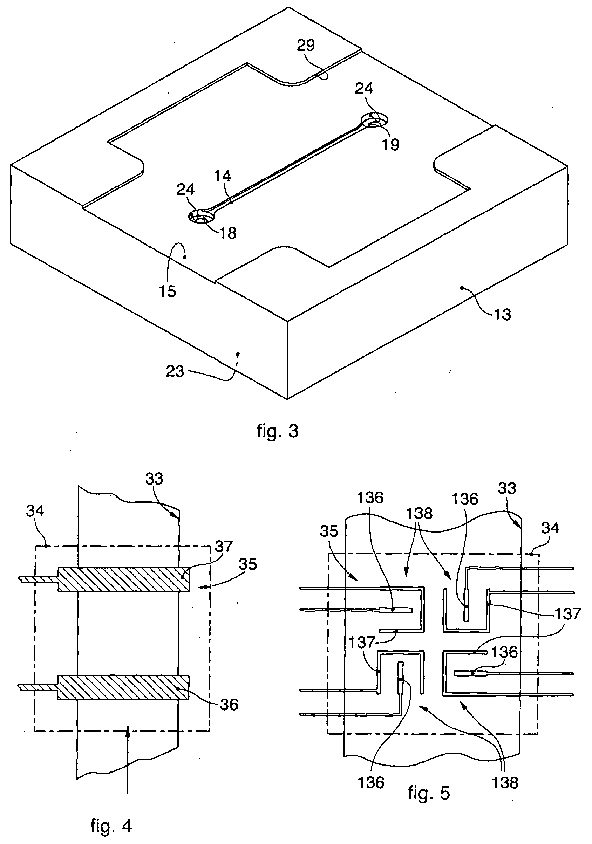

The first surface 15 (figs. 2 and 3) is defined by a shaped housing seating 29 made recessed in the overall thickness of the base body 13.

The housing seating 29 is configured to allow the stable positioning and accurate housing of a cover element, in this case a slide 30 (fig. 1), which is put in contact with a supporting surface 31 against the first surface 15 of the base body 13 and provides to close the microgroove 14 so as to define a micro-channel 33 for the passage of the blood.

The first surface 15 of the base body 13 and the support surface 31 of the slide 30 have very strict geometrical and dimensional tolerances, to guarantee the correct reciprocal planarity between them, and to guarantee the hermetic closure of the micro-channel 33.

The syringe pump 27 generates a depression in the micro-channel 33 which not only aspirates the blood from the containing element 28 but also maintains the base body 13 and the slide 30 adherent with respect to each other.

On the support surface 31 of the slide 30, before analysis, a cyto-adhesive substance is uniformly distributed, such as a collagen, to promote the platelet clustering of the blood.

Attachment means of a known type, not shown in the drawings, are provided to reciprocally attach the base body 13 and the slide 30.

The perfusion chamber 11 is typically made of transparent material to allow the acquisition of images by the optical acquisition means. Its geometry must be suitable to simulate the desired fluid-dynamic conditions, such as for example the degree of sliding or shear rate and the laminar flow.

In particular, the perfusion chamber 11 must guarantee a good hydraulic seal, in order to preserve the desired fluid-dynamic conditions and not to affect the reliability of the analysis.

The material used to make the perfusion chamber 11, that is, for the base body 13 and the slide 30, is chosen from a group comprising glass, COC (Cyclic Olefin Copolymers), COP (Cyclic Olefin Polymers), high optical grade polycarbonate, casting silicone, or polydimethylsiloxane, also known as PDMS, or suchlike.

On the support surface 31 of the slide 30 and in investigation zones 34, in this case two investigation zones 34, of the same support surface 31 which is disposed in correspondence with the micro-channel 33, there are impedance assessment means 35 (fig. 4), which perform an impedance assessment of the platelet cluster.

Merely by way of example, each investigation zone 34 is about 0.2 mm wide and 0.2 mm long.

In a first form of embodiment (fig. 4), the impedance assessment means 35 comprise a first electrode 36 and a second electrode 37 both having an oblong development and disposed parallel to each other, transverse to the longitudinal development of the micro-channel 33.

Merely by way of example, the first electrode 36 and second electrode 37, about 250 μm long, are disposed parallel to each other and reciprocally distanced by about 200 μm.

In a second form of embodiment (fig. 5), the impedance assessment means 35 comprise four elementary measuring cells 138, each rotated by 90° with respect to the adjacent other two elementary measuring cells 138.

Each investigation zone 34 is therefore divided into a plurality of investigation portions to each of which, in turn, an elementary measuring cell 138 refers.

Each elementary measuring cell 138 comprises a first electrode 136, central, surrounded by a second electrode 137 which is U-shaped, that is, having three rectilinear segments reciprocally connected to each other at the respective ends and disposed angled with respect to each other by an angle of 90°.

Merely by way of example the first electrode 136 is about 80 μm long and 20 μm wide, while the second electrode 137 is about 10 μm wide.

In both forms of embodiment described with reference to fig. 4 and fig. 5, the first electrodes 36, 136 and the second electrodes 37, 137 are made of conductive material chosen from at least gold, platinum and indium-tin oxide, or tin-doped indium oxide, also known by the acronym ITO.

The first electrodes 36, 136 and the second electrodes 37, 137 are made by molding on the support surface 31, for example using physical vapor deposition (PVD) techniques, sputtering techniques or other similar or comparable techniques.

The first electrodes 36, 136 and the second electrodes 37, 137 comprise an active sensitive part which during use is in direct contact with the blood, and the remaining part of the electrode which is insulated, for example using passivization techniques.

During the measurements, each pair of first 36, 136 and second electrodes 37, 137 are subjected, independently of each other, to a difference in potential and the current in transit is detected.

In particular, the first electrodes 36, 136 and the second electrodes 37, 137 are associated, independently of each other, with electric feed devices 40 of a known type, and with devices 41 to measure the electrical quantities.

The optical acquisition device 12 comprises an optical module 42 for epifluorescence analysis, to highlight the platelets that are marked with a suitable fluorescent probe to adhere to the cyto-adhesive substrate.

The optical module 42 can be the fluorescence type with or without lenses of the two-dimensional type.

The electrical feed devices 40, the measurement devices 41 and the optical acquisition devices are associated with a processing unit 43 provided to control, command, acquire data, and process data supplied by said devices.

The apparatus 10 according to the present invention may advantageously comprise thermostat means, to keep the perfusion chamber 11 at a desired temperature, advantageously the physiological temperature which in the case of the human body is about 37°C.

The method to analyze the process of platelet clustering using an apparatus 10 as described above will now be described.

The method comprises at least a step in which a sample of hematic fluid is taken from a patient and suitably prepared in various ways, which vary according to the tests to be carried out.

More specifically, at least a sample of venous blood is required, the blood is temporarily preserved in a test tube containing anti-coagulant substances such as for example heparin, citrate or suchlike, and fluorescent substances, for example quinacrine and anti-fibrin antibody phycoerythrin are added.

Then, a cyto-adhesive substance is deposited on the support surface 31 of the slide 30, and the slide 30 is then positioned on the base body 13.

Subsequently, the blood is circulated through the micro-channel 33 by driving the syringe pump 27, in order to start the phenomenon of platelet clustering which, by way of example only, can last about 6 minutes.

At this point a step is started of analyzing the process of platelet clustering, which provides at least an optical measurement substep, an impedance assessment substep, and a processing substep during which the information acquired in the previous two substeps is correlated.

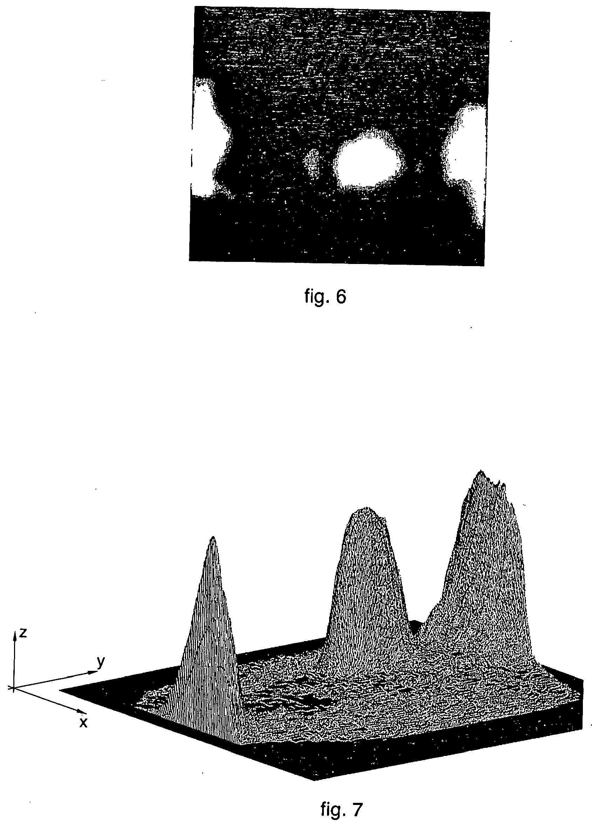

The optical measurement substep allows to estimate over time, and using the optical acquisition device 12, the spatial distribution of a platelet cluster on the plane of the slide 30, and in the investigation zone 34. In particular, during the optical measurement substep, images are acquired over time, one of which is shown in fig. 6, and a base area is estimated, that is, the plan surface evaluated by the optical acquisition device 12 located above the investigation zones 34, of a platelet cluster adhering inside each investigation zone 34 at the instant considered.

The impedance assessment substep allows to determine over time the evolution of the average height of the platelet cluster inside each of the investigation zones 34.

In order to carry out the impedance assessments, it was necessary to characterize the electrical behavior of the plasma, blood and platelet cluster. Blood is formed mainly of plasma, red corpuscles, white corpuscles and platelets. From the electrical point of view plasma behaves like a saline solution and hence mainly as a conductor (sigma 1 S/m); on the contrary, the cell content is formed by a lipid membrane (a dielectric) containing an intracellular fluid called cytoplasm. There is an optimum range of frequencies in which the impedance assessments are carried out; at low frequencies the double layer phenomenon is mainly measured, due to the reaction to the electrodes of the salts dissolved in the plasma (the measurement of the double layer does not give any information except that connected to the electrode-plasma interface), while at high frequencies the membrane of the cells no longer behaves as a dielectric and in practice a measurement is carried out on the cytoplasm.

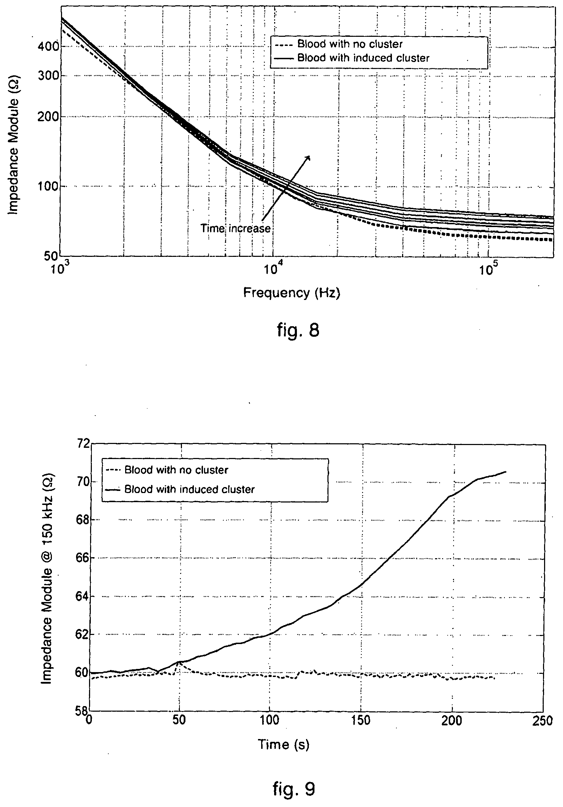

Fig. 8 shows two families of curves relating to the spectroscopy of impedance on blood without induction of thrombus and with induction of thrombus. As can be seen, in both families of curves the left part of the graph (low frequency) has a decreasing behavior in frequency, which is due to the phenomenon of interface of the double layer; instead, at higher frequencies the phenomenon becomes resistive and therefore the measurement must be carried out at frequencies comprised between 100 kHz and 1 MHz.

It can also be seen that, in the absence of platelet clustering (curves shown by dashes) the curves all overlap (no change in impedance), whereas in the presence of clustering (continuous curves), the family of curves evolves monotonously, increasing the impedance value read. Fig. 9 shows the temporal evolution of the impedance evaluated at a frequency of 150 kHz.

It can be seen that, without inducing platelet clustering, the impedance value remains constant over time, whereas by inducing the thrombotic formation there is a considerable increase in the measurement, in this case after 50 seconds.

Another factor to be considered for measuring the phenomenon of clustering concerns the amplitude of the electrical signals involved in relation to the materials chosen to make the electrodes; with gold electrodes the amplitude of the signal injected is less than 1 volt so as not to compromise the linearity of the phenomenon.

With reference to the measurements made in the subsequent forms of embodiment, the signal injected was chosen with an amplitude of 0.1 volt.

During the processing substep the volume of the platelet cluster is estimated over time in each of the investigation zones 34. In particular, from the measurement of the impedance, the base area determined optically and the luminance emitted by the platelet thrombus, it is possible to effect an inversion and find the overall volume of the thrombus that has formed inside the investigation area 34.

The volume of the platelet cluster for each investigation area 34 is determined by correlating the base area "S" of the platelet cluster and the height of the platelet cluster, both evaluated as a function of the time "t".

The base area S is evaluated as a function of the images acquired during the optical measurement substep.

The height of the cluster is evaluated as a function of the impedance assessments made.

The acquisition of the images allows to construct a shape function, for example of the type shown in fig. 7, the projection of which on a Cartesian plane (x, y) of the slide 30 coincides with the two-dimensional optical image acquired in the given instant of time. From the image it is possible to determine for each point or pixel of the image a value of light intensity, or luminance. The shape function therefore has a third dimension "z", function of the time and the point considered which is directly proportional to the light intensity detected from the image for that given point.

The third dimension "z" of the shape function is expressed by the formula:

$$z(x, y, t) = k(t) \, I(x, y, t)$$

that is, the coordinate $z(x, y, t)$ of point $(x, y)$ (or pixel of the image acquired) is proportional to the light intensity $I(x, y, t)$ of the point or pixel considered $(x, y)$, where $k(t)$ is an unknown proportionality constant in a given instant of time $t$.

The proportionality constant $k(t)$ is determined by using a 3D simulation of the electro-quasistatic type on the same geometry as the elementary measuring cell using the Discreet Geometric Approach on a cubic, tetrahedral or polyhedral grid, so as to reproduce with minimal discrepancy the value of impedance measured at the terminals of the electrodes at a frequency of $f < 200$ kHz.

During this simulation, an inversion technique is used which provides to modulate the value of the parameter $k(t)$ until the function $z(x, y, t)$ is found, which best approximates the development of the impedance measurements. From this function it is possible to immediately calculate the equivalent volume $V(t)$ of the cluster, by integrating the function $z(x, y, t)$ suitably scaled.

With reference to the form of embodiment described with reference to fig. 5, the elementary measuring cell 138 has an electrical behavior of the anisotropic type, given that the current density is mainly directed in a direction perpendicular to the central electrode.

Therefore, the impedance measured depends on that direction and is sensitive to the increase in height $z(x, y, t)$ of the platelet cluster along points $(x, y)$ of said direction at instant $t$. For each elementary measuring cell 138 a shape function $z(x, y, t)$ is determined, from which it is possible to identify an average height which is associated to the cell and relating to one direction.

In this way, from the four average heights obtained for each elementary measuring cell 138, it is possible to determine the average height h of the investigation zone 34 to which the four elementary measuring cells 138 belong.

The particular disposition of the elementary measuring cells 138 inside the investigation zone 34 allows to investigate the growth in height of the shape function $z(x, y, t)$ according to directions that are orthogonal to each other.

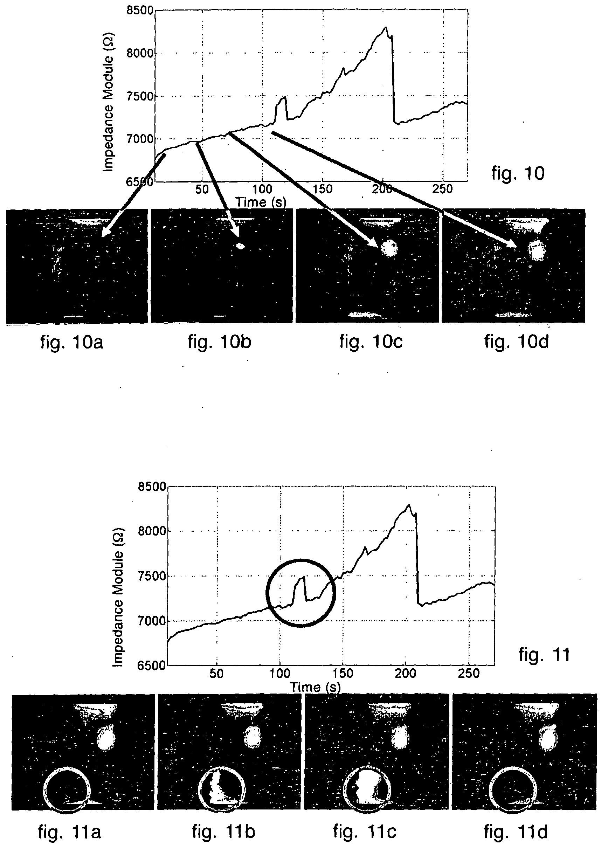

Fig. 10 shows the development of the impedance detected during the impedance assessment substep in a first initial phase, in which there is a slow growth of the thrombus, compared with the image acquisitions made in the optical measurement substep (figs. 10a, 10b, 10c, 10d).

After this initial phase, a platelet cluster, formed outside the visual field due to the effect of the flow, is transported inside the analysis zone for a few seconds and then swept away again (figs. 11, 11a, 11b, 11c, lid).

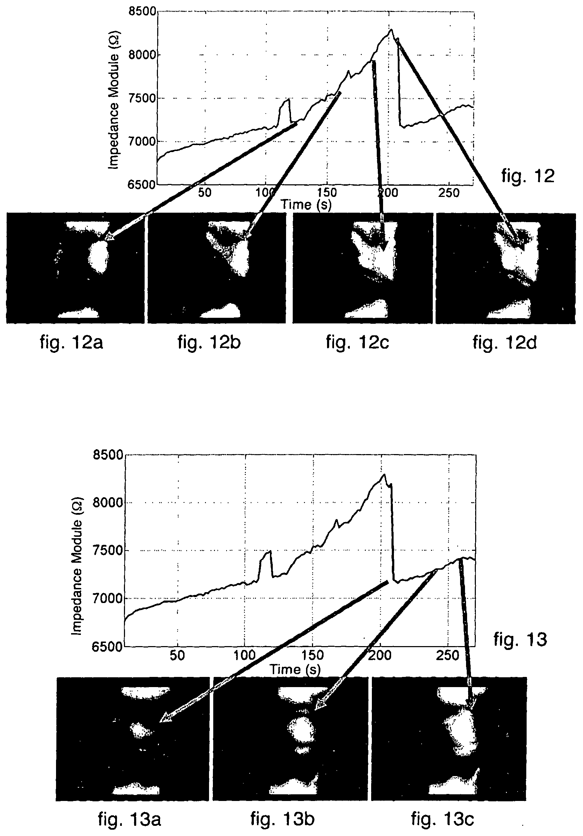

There is then a third phase in which there is a rapid growth in volume of the platelet cluster inside the analysis area (figs. 12, 12a, 12b, 12c, 12d).

There is finally a last phase in which the thrombus, having a considerable volume, is swept away by the flow of blood and at that point new clusters are again formed (figs. 13, 13a, 13b, 13c).

**Claims**

1. Method to analyze the cluster formation process in a biological fluid, such as blood or hematic fluids, comprising at least a step in which said biological fluid is made to flow through at least a micro-channel (33) of a perfusion chamber (11), there being a reactive substrate, such as a cyto-adhesive substrate, present in said micro-channel (33) to stimulate a clustering process in said biological fluid, and an optical acquisition step using an optical acquisition device (12), during which two-dimensional images relating to the formation of clusters are acquired in at least one investigation area (34) of said micro-channel (33), **characterized in that** it comprises

   - an impedance assessment step associated to said optical acquisition step, during which, by means of assessment means (35) disposed in correspondence to said investigation area (34), a measurement is carried out over time of the impedance of said biological fluid, and
   - a processing step during which, starting from said two-dimensional images, a shape function is constructed, the projection of which on a Cartesian plane $(x, y)$ coincides with the two-dimensional optical image acquired in the given instant of time, in which said shape function has a third dimension $z(x, y, t) = k(t)I(x, y, t)$, where $I(x, y, t)$ is the light intensity evaluated in the time "t" for every point "x, y" of the image, and $k(t)$ is a proportionality constant, and in which, by means of integration of said shape function, the volume of clusters forming in said investigation area (34) is determined, correlating over time the development of said third dimension $z(x, y, t)$ with the development of the impedance acquired in said impedance assessment step.

2. Method as in claim 1, **characterized in that,** in said processing step and from said images, a base area (S) is estimated of the clusters which form in said investigation area (34), **and in that** said third dimension $z(x, y, t)$ is processed, as a function of said base area (S), so as to approximate it to the development of the impedance measurements acquired in said impedance assessment step and to determine the volume of said cluster.

3. Method as in claim 2, **characterized in that** said base area (S) of the clusters is determined as a function of the light intensity I(x, y, t) measured from said images.

4. Method as in any claim hereinbefore, **characterized in that** said proportionality constant k(t) is determined using a 3D simulation of the electro-quasistatic type using the Discreet Geometric Approach on a cubic, tetrahedral or polyhedral grid, in order to reproduce the development of the impedance measurements.

5. Method as in any claim hereinbefore, **characterized in that** during said processing step an inversion technique is used which provides to modulate the value of the proportionality constant k(t) so as to identify said dimension z(x, y, t) with a development that can be approximated with that of the impedance measurements detected in said impedance assessment step.

6. Apparatus to analyze the cluster formation process in a biological fluid, such as blood or hematic fluids, comprising a perfusion chamber (11), provided with at least a micro-channel (33) through which said biological fluid is able to flow, and in which at least a reactive substrate is present, such as a cyto-adhesive substrate, to stimulate the clustering process in the biological fluid, and an optical acquisition device (12) suitable to acquire two-dimensional images relating to the formation of clusters in at least one investigation area (34) of said micro-channel (33), **characterized in that** it comprises impedance assessment means (35) associated to said micro-channel (33), in correspondence to said investigation area (34) and disposed, during use, in contact with the stream of the biological fluid passing through, to measure impedance data over time of said biological fluid correlated to the formation of clusters, and a processing unit (43) configured to construct, starting from said two-dimensional images, a shape function the projection of which on a Cartesian plane (x,y) coincides with the two-dimensional optical image acquired in the given instant of time, in which said shape function has a third dimension z(x, y, t) = k(t)I(x, y, t), where I(x, y, t) is the light intensity evaluated in the time "t" for every point "x, y" of the image, and k(t) is a proportionality constant, in which said processing unit is configured to determine the volume of clusters forming in said micro-channel (33), by means of a correlation, over time, of the development of said third dimension z(x, y, t) with the development detected by said impedance detection means (35).

7. Apparatus as in claim 6, **characterized in that** said impedance assessment means (35) comprise electrodes (36, 37; 136, 137) to which measuring devices (41) are associated in order to measure said impedance data.

8. Apparatus as in claim 7, **characterized in that** said electrodes (36, 37; 136, 137) are made of conductive material chosen from a group comprising at least gold, platinum, indium-tin oxide.

9. Apparatus as in claim 7 or 8, **characterized in that** said impedance assessment means (35) comprise at least a first electrode (36) and a second electrode (37) having an oblong development and disposed, parallel with respect to each other, transverse to the longitudinal development of the micro-channel (33), said investigation area (34) being comprised between said first electrode (36) and said second electrode (37).

10. Apparatus as in claim 7 or 8, **characterized in that** said impedance assessment means (35) comprise a plurality of elementary measuring cells (138) angularly offset with respect to each other and each provided with at least a pair of said electrodes (136, 137).

11. Apparatus as in claim 10, **characterized in that** each of said elementary measuring cells (138) comprises a first central electrode (136) surrounded by a second electrode (137) which is U-shaped.

12. Apparatus as in any claim from 6 to 11, **characterized in that** said optical acquisition device (12) comprises an optical module (42) of the fluorescent type with or without lenses of the two-dimensional type.

**Patentansprüche**

1. Verfahren zur Analyse des Prozesses der Clusterbildung in einem biologischen Fluid, wie z. B. Blut oder hämatische Flüssigkeiten, das mindestens einen Schritt beinhaltet, bei welchem besagtes biologisches Fluid dazu gebracht wird, durch mindestens einen Mikrokanal (33) einer Perfusionskammer (11) zu fließen, wo sich in besagtem Mikrokanal (33) ein reaktives Substrat befindet, wie z. B. ein cytoadhäsives Substrat, um einen Clusteringprozess in besagtem biologischen Fluid anzuregen, und einen Schritt der optischen Erfassung mittels eines optischen Erfassungsgerätes (12), während dem zweidimensionale Bilder im Zusammenhang mit der Bildung von Clustern in mindestens einem Untersuchungsbereich (34) von besagtem Mikrokanal (33) erfasst werden, **dadurch gekennzeichnet, dass** es aufweist

    - einen Impedanzbewertungsschritt, der mit besagtem Schritt der optischen Erfassung zusammengehört, während dem mit Hilfe von Bewertungsmitteln (35), die entsprechend zu besagtem Untersuchungsbereich (34) angeordnet sind, eine Messung der Impedanz von besagtem biologischen Fluid über die Zeit durchgeführt wird, und

- ein Verarbeitungsschritt, während dem, ausgehend von besagten zweidimensionalen Bildern, eine Formfunktion konstruiert wird, deren Projektion auf eine kartesische Ebene (x,y) mit dem zu gegebenem Zeitpunkt erfassten zweidimensionalen optischen Bild übereinstimmt, wobei besagte Formfunktion eine dritte Dimension $z(x, y, t) = k(t) I(x, y, t)$ hat, wobei $I(x, y, t)$ die Lichtintensität ist, die für jeden Punkt "x, y" des Bildes zur Zeit "t" ausgewertet ist und $k(t)$ eine Proportionalitätskonstante ist, und wobei mittels Integration von besagter Formfunktion das Volumen von Clustern, die sich in besagtem Untersuchungsbereich (34) bilden, bestimmt wird, wobei die Entwicklung besagter dritter Dimension $z(x, y, t)$ über die Zeit mit der Entwicklung der Impedanz korreliert, welche in besagtem Impedanzbewertungsschritt erfasst wurde.

2. Verfahren gemäß Anspruch 1, **dadurch gekennzeichnet, dass** in besagtem Verarbeitungsschritt und von besagten Bildern eine Grundfläche (S) der Cluster, die sich in besagtem Untersuchungsbereich (34) bilden, abgeschätzt wird, und dadurch, dass besagte dritte Dimension $z(x, y, t)$ als Funktion von besagter Grundfläche (S) so verarbeitet wird, dass sie an die Entwicklung der Impedanzmessungen angenähert wird, welche in besagtem Impedanzbewertungsschritt erfasst wurden, und dass das Volumen von besagtem Cluster bestimmt wird.

3. Verfahren gemäß Anspruch 2, **dadurch gekennzeichnet, dass** besagte Grundfläche (S) der Cluster als eine Funktion der Lichtintensität $I(x, y, t)$, die von besagten Bildern gemessenen wurde, bestimmt wird.

4. Verfahren gemäß einem der vorangehenden Ansprüche, **dadurch gekennzeichnet, dass** besagte Proportionalitätskonstante $k(t)$ unter Verwendung einer 3D-Simulation der elektro-quasistatischen Art, welche den diskreten geometrischen Ansatz auf einem kubischen, tetraedrischen oder polyedrischen Gitter anwendet, bestimmt wird, um die Entwicklung der Impedanzmessung zu reproduzieren.

5. Verfahren gemäß einem der vorangehenden Ansprüche, **dadurch gekennzeichnet, dass** während besagtem Verarbeitungsschritt eine Inversionstechnik angewendet wird, welche vorsieht, den Wert der Proportionalitätskonstanten $k(t)$ so anzupassen, dass besagte Dimension $z(x, y, t)$ mit einer Entwicklung identifiziert wird, die durch die der Impedanzmessung angenähert werden kann, welche in besagtem Impedanzbewertungsschritt detektiert wurde.

6. Gerät zur Analyse des Prozesses der Clusterbildung in einem biologischen Fluid, wie z. B. Blut oder hämatische Flüssigkeiten, das eine Perfusionskammer (11) aufweist, welche mit mindestens einem Mikrokanal (33) ausgestattet ist, durch welchen besagtes biologisches Fluid fließen kann, und in welcher sich mindestens ein reaktives Substrat befindet, wie z. B. ein cytoadhäsives Substrat, um den Clusteringprozess in dem biologischen Fluid anzuregen, und ein optisches Erfassungsgerät (12), das geeignet ist, um zweidimensionale Bilder im Zusammenhang mit der Bildung von Clustern in mindestens einem Untersuchungsbereich (34) von besagtem Mikrokanal (33) zu erfassen, **dadurch gekennzeichnet, dass** es Impedanzbewertungsmittel (35) aufweist, die besagtem Mikrokanal (33) zugeordnet sind, entsprechend zu besagtem Untersuchungsbereich (34) und bei Gebrauch in Kontakt mit dem durchlaufenden Strom biologischen Fluids angeordnet sind, um Impedanzdaten von besagtem biologischen Fluid über die Zeit zu messen, welche mit der Bildung von Clustern korrelieren, und eine Verarbeitungseinheit (43), die gestaltet ist, um ausgehend von besagten zweidimensionalen Bildern eine Formfunktion zu konstruieren, deren Projektion auf eine kartesische Ebene (x, y) mit dem zu gegebenem Zeitpunkt erfassten zweidimensionalen optischen Bild übereinstimmt, wobei besagte Formfunktion eine dritte Dimension $z(x, y, t) = k(t) I(x, y, t)$ hat, wobei $I(x, y, t)$ die Lichtintensität ist, die zur Zeit "t" für jeden Punkt "x, y" des Bildes ausgewertet ist, und $k(t)$ eine Proportionalitätskonstante ist, wobei besagte Verarbeitungseinheit gestaltet ist, um das Volumen von Clustern zu bestimmen, die sich in besagtem Mikrokanal (33) bilden, und zwar mittels einer Korrelation über die Zeit der Entwicklung von besagter dritter Dimension $z(x, y, t)$ mit der Entwicklung, die durch besagte Impedanzbewertungsmittel (35) erfasst wurde.

7. Gerät gemäß Anspruch 6, **dadurch gekennzeichnet, dass** besagte Impedanzbewertungsmittel (35) Elektroden (36, 37; 136, 137) aufweisen, zu denen Meßgeräte (41) gehören, um besagte Impedanzdaten zu messen.

8. Gerät gemäß Anspruch 7, **dadurch gekennzeichnet, dass** besagte Elektroden (36, 37; 136, 137) aus leitendem Material sind, das aus einer Gruppe ausgewählt wurde, die mindestens Gold, Platin, Indiumzinnoxid aufweist.

9. Gerät gemäß Anspruch 7 oder 8, **dadurch gekenn-**

**zeichnet, dass** besagte Impedanzbewertungsmittel (35) mindestens eine erste Elektrode (36) und eine zweite Elektrode (37) aufweisen, die einen rechteckigen Aufbau haben und, selbst zueinander parallel, quer zum Längsaufbau des Mikrokanals (33) angeordnet sind, wobei besagter Untersuchungsbereich (34) den Bereich zwischen besagter erster Elektrode (36) und besagter zweiten Elektrode (37) aufweist.

10. Gerät gemäß Anspruch 7 oder 8, **dadurch gekennzeichnet, dass** besagte Impedanzbewertungsmittel (35) eine Vielzahl von elementaren Messzellen (138) aufweisen, die einen Winkelversatz zueinander aufweisen und von denen jede mit mindestens einem Paar besagter Elektroden (136, 137) ausgestattet ist.

11. Gerät gemäß Anspruch 10, **dadurch gekennzeichnet, dass** jede der besagten elementaren Messzellen (138) eine erste zentrale Elektrode (136) aufweist, die von einer zweiten Elektrode (137) umgeben ist, welche u-förmig ist.

12. Gerät gemäß einem der Ansprüche 6 bis 11, **dadurch gekennzeichnet, dass** besagtes optisches Erfassungsgerät (12) ein optisches Modul (42) des Fluoreszenstyps mit oder ohne Linsen der zweidimensionalen Art aufweist.

**Revendications**

1. Procédé pour analyser le processus de formation d'amas dans un fluide biologique, tel que le sang ou des fluides hématiques, comprenant au moins une étape dans laquelle le fluide biologique est amené à s'écouler à travers au moins un micro-canal (33) d'une chambre de perfusion (11), un substrat réactif, tel qu'un substrat cyto-adhésif, étant présent dans ledit micro-canal (33) pour stimuler un processus de formation d'amas dans ledit fluide biologique, et une étape d'acquisition optique utilisant un dispositif d'acquisition optique (12), durant laquelle des images bidimensionnelles concernant la formation d'amas sont acquises dans au moins une zone d'investigation (34) dudit micro-canal (33), **caractérisé en ce qu**'il comprend

    - une étape d'évaluation d'impédance associée à ladite étape d'acquisition optique, durant laquelle, au moyen d'un moyen d'évaluation (35) disposé au niveau de ladite zone d'investigation (34), une mesure de l'impédance du fluide biologique est réalisée au cours du temps, et
    - une étape de traitement durant laquelle, à partir desdites images bidimensionnelles, une fonction de forme est définie, dont la projection sur un plan cartésien (x, y) coïncide avec l'image optique bidimensionnelle acquise dans l'instant de temps donné, dans lequel ladite fonction de forme comporte une troisième dimension z(x, y, t) = k(t)I(x, y, t), où I(x, y, t) est l'intensité lumineuse évaluée dans le temps "t" pour chaque point "x, y" de l'image, et k(t) est une constante de proportionnalité, et dans lequel, par l'intégration de ladite fonction de forme, le volume des amas qui se forment dans ladite zone d'investigation (34) est déterminé, corrélant, au cours du temps, le développement de ladite troisième dimension z (x, y, t) avec le développement de l'impédance acquise dans ladite étape d'évaluation d'impédance.

2. Procédé selon la revendication 1, **caractérisé en ce que,** dans ladite étape de traitement et à partir desdites images, une aire de base (S) des amas qui se forment dans ladite zone d'investigation (34) est estimée, et dans lequel ladite troisième dimension z(x, y, t) est traitée en fonction de ladite aire de base (S), de manière à l'approximer au développement des mesures d'impédance acquises dans ladite étape d'évaluation d'impédance et à déterminer le volume dudit amas.

3. Procédé selon la revendication 2, **caractérisé en ce que** ladite aire de base (S) des amas est déterminée en fonction de l'intensité lumineuse I (x, y, t) mesurée à partir desdites images.

4. Procédé selon n'importe laquelle des revendications précédentes, **caractérisé en ce que** ladite constante de proportionnalité k(t) est déterminée au moyen d'une simulation 3D de type électroquasistatique utilisant l'approche géométrique discrète sur une grille cubique, tétraédrique ou polyédrique, pour reproduire le développement des mesures d'impédance.

5. Procédé selon n'importe laquelle des revendications précédentes, **caractérisé en ce que** durant ladite étape de traitement une technique d'inversion est utilisée, qui permet de moduler la valeur de la constante de proportionnalité k(t) de manière à identifier ladite dimension z(x, y, t) avec un développement approximable au développement des mesures d'impédance détectées ans ladite étape d'évaluation d'impédance.

6. Appareil pour analyser le processus de formation d'amas dans un fluide biologique, tel que le sang ou des fluides hématiques, comprenant une chambre de perfusion (11) pourvue d'au moins un micro-canal (33) à travers lequel peut s'écouler ledit fluide biologique, et dans lequel est présent au moins un substrat réactif, tel qu'un substrat cyto-adhésif, pour stimuler le processus de formation d'amas dans le fluide biologique, et un dispositif d'acquisition optique (12), conçu pour acquérir des images bidimensionnelles concernant la formation d'amas dans au moins une zone d'investigation (34) dudit micro-canal (33), **caractérisé en ce qu**'il comprend des moyens d'évaluation d'impédance (35) associés audit micro-canal (33) au niveau de ladite zone d'investigation (34), et disposés, pendant l'emploi, en contact avec le flux de fluide biologique les traversant, pour mesurer des données d'impédance dudit fluide biologique au cours du temps en corrélation avec la formation d'amas, et une unité de traitement (43) configurée pour réaliser, à partir desdites images bidimensionnelles, une fonction de forme dont la projection sur un plan cartésien (x, y) coïncide avec l'image optique bidimensionnelle acquise dans l'instant de temps donné, dans lequel ladite fonction de forme comporte une troisième dimension z(x, y, t) = k(t)I(x, y, t), où I(x, y, t) est l'intensité lumineuse évaluée dans le temps "t" pour chaque point "x, y" de l'image, et k(t) est une constante de proportionnalité, dans lequel ladite unité de traitement est configurée pour déterminer le volume d'amas qui se forment dans ledit micro-canal (33) au moyen d'une corrélation, au cours du temps, du développement de ladite troisième dimension z (x, y, t) avec le développement détecté par lesdits moyens d'évaluation d'impédance (35).

7. Appareil selon la revendication 6, **caractérisé en ce que** lesdits moyens d'évaluation d'impédance (35) comprennent des électrodes (36, 37 ; 136, 137) auxquelles sont associés des dispositifs de mesure (41) pour mesurer lesdites données d'impédance.

8. Appareil selon la revendication 7, **caractérisé en ce que** lesdites électrodes (36, 37 ; 136, 137) sont constituées avec un matériau conducteur choisi dans un groupe comprenant au moins l'or, le platine, l'oxyde d'indium-étain.

9. Appareil selon la revendication 7 ou 8, **caractérisé en ce que** lesdits moyens d'évaluation d'impédance (35) comprennent au moins une première électrode (36) et une seconde électrode (37) ayant un développement oblong et disposées en parallèle entre elles, transversalement au développement longitudinal du micro-canal (33), ladite zone d'investigation (34) étant comprise entre ladite première électrode (36) et ladite seconde électrode (37).

10. Appareil selon la revendication 7 ou 8, **caractérisé en ce que** lesdits moyens d'évaluation d'impédance (35) comprennent une pluralité de cellules de mesure élémentaires (138), en décalage angulaire les unes par rapport aux autres, et chacune pourvue d'au moins une paire desdites électrodes (136, 137).

11. Appareil selon la revendication 10, **caractérisé en ce que** chacune desdites cellules de mesure élémentaires (138) comprend une première électrode centrale (136) entourée par une seconde électrode (137) en U.

12. Appareil selon l'importe laquelle des revendications 6 à 11, **caractérisé en ce que** ledit dispositif d'acquisition optique (12) comprend un module optique (42) du type à fluorescence avec ou sans lentilles de type bydimensionnel.

fig. 1

fig. 2

fig. 3

fig. 4

fig. 5

fig. 6

fig. 7

fig. 8

fig. 9

17

fig. 10

fig. 10a      fig. 10b      fig. 10c      fig. 10d

fig. 11

fig. 11a      fig. 11b      fig. 11c      fig. 11d

fig. 12

fig. 12a     fig. 12b     fig. 12c     fig. 12d

fig. 13

fig. 13a     fig. 13b     fig. 13c

**EP 2 845 002 B1**

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- EP 2010903 A
- US 20060211071 A
- KR 2008015212
- SU 1278697
- US 6797150 B2
- US 20080297169 A1
- WO 2005114140 A1
- WO 2011073481 A1

## Non-patent literature cited in the description

- **M. MAZZUCATO ; M. R. COZZI ; P. PRADELLA ; D. PERISSINOTTO ; A. MALMSTROM ; M. MORGELIN ; P. SPESSOTTO ; A. COLOMBATTI ; L. DE MARCO ; R. PERRIS, VASCULAR.** PG-M/versican variants promote platelet adhesion at low shear rates and cooperate with collagens to induce aggregation. *FASEB J.,* 2002, vol. 16, 1903-1916
- **M. MAZZUCATO ; P. PRADELLA ; M.R. COZZI ; L. DE MARCO ; Z. M. RUGGERI.** Sequential cytoplasmic calcium signals in a 2-stage platelet activation process induced by the glycoprotein Ibalpha mechanoreceptor. *Blood,* 2002, vol. 100, 2793-2800
- **M. MAZZUCATO ; M. R. COZZI ; P. PRADELLA ; Z. M. RUGGERI ; L. DE MARCO.** Distinct roles of ADP receptors in von Willebrand factormediated platelet signaling and activation under high flow. *Blood,* 2004, vol. 104, 3221-3227
- **A. CASONATO ; L. DE MARCO ; L. GALLINARO ; M. SZTUKOWSKA ; M. MAZZUCCATO ; M. BATTISTON ; A. PAGNAN ; Z.M. RUGGERI.** Altered von Willebrand factor subunit proteolysis and multimer processing associated with the Cys2362Phe mutation in the B2 domain. *Thrombosis and Haemostasis,* 2007, vol. 97, 527-533
- **M. MAZZUCATO ; M. R. COZZI ; M. BATTISTON ; M. JANDROT-PERRUS ; M. MONGIAT ; P. MARCHESE ; T. J. KUNICKI ; Z. M. RUGGERI ; L. DE MARCO.** Distinct spatio-temporal Ca2+ signaling elicited by integrin alpha2β1 and glycoprotein V1 under flow. *Blood,* 2009, vol. 114, 2793-280
- **U. GURKAN ; S. MOON ; H. GECKI ; F. XU ; S. WANG ; T. J. LU ; U. DEMIRCI.** Miniaturized lensless imaging systems for cell and microorganism visualization in point-of-care testing. *Biotechnol. J.,* 2011, vol. 6, 138-149
- **D. C. CARDINAL ; R. J. FLOWER.** The electronic aggregometer: A novel device for assessing platelet behavior in blood. *J. Pharmacol. Methods,* 1980, vol. 3, 135-158
- **T. DAI ; A. ADLER.** In vivo blood characterization from bioimpedance spectroscopy of blood pooling. *IEEE Trans. Instr. Meas.,* 2009, vol. 58, 3831-3838
- **G.A.M. POP ; T.L.M. DE BACKER ; M. DE JONG ; P.C. STRUIJK ; L. MORARU ; Z. CHANG ; H. G. GOOVAERTS ; C. J. SLAGER ; A. J. J. C. BOGERS.** On-line electrical impedance measurement for monitoring blood viscosity during on-pump heart surgery. *Eur. Surgical Res.,* 2004, vol. 36, 259-265
- **Y. ULGEN ; M. SEZDI.** Physiological quality assessment of stored whole blood by means of electric measurements. *Med. Bio. Eng. Comput.,* 2007, vol. 45, 653-660
- **Y. ULGEN ; M. SEZDI.** Hematocrit dependence of the Cole-Cole parameters of human blood. *IEEE Proc. 2nd Int. Biomed. Eng. Days,* 1998, 71-74
- Investigation of electrical resistivity changes of human blood during dynamic exercise. **T. YAMAGATA ; H. FUJISAKI.** Trans. Electrical and Electronic Engineering. IEEJ, 2008, vol. 3, 79-83
- **A. E. HOETINK ; T. J. C. FAES ; K. R. VISSER ; R. M. HEETHAAR.** On the flow dependency of the electrical conductivity of blood. *IEEE Trans. Biomed. Eng.,* 2004, vol. 51, 1251-1261
- **R. L. GAW ; B. H. CORNISH ; B. J. THOMAS.** The Electrical Impedance of Pulsatile Blood Flowing Through Rigid Tubes: A Theoretical Investigation. *IEEE Trans. Biomed. Eng.,* 2008, vol. 55, 721-727
- **K. ASAMI ; K. SEKINE.** Dielectric modelling of erythrocyte aggregation in blood. *J. Phys. D: Appl. Phys.,* 2007, vol. 40, 21972204
- **O. BASKURT ; M. UYUKLU ; S. OZDEM ; H. J. MEISELMAN.** Measurement of red blood cell aggregation in disposable capillary tubes. *Clinical Hemorheology and Microcirculation,* 2011, vol. 47, 295-305

- **O. BASKURT ; M. UYUKLU ; H. J. MEISELMAN.** Simultaneous monitoring of electrical conductance and light transmittance during red blood cell aggregation. *Biorheology,* 2009, vol. 46, 239-249
- **O. KWON ; J. K. SEO ; E. J. WOO ; J-R. YOON.** Electrical Impedance Imaging for Searching anomalies. *Comm. Korean Math. Soc.,* 2001, vol. 16 (3), 459-485
- **N. PIACENTINI ; D. DEMARCHI ; P. CIVERA ; M. KNAFLITZ.** Blood Cell Counting by Means of Impedance Measurements in a Microsystem Device. *Proc. 30th Int. IEEE EMBS Conf.,* 2008, 4824-4827
- **Y. NAKASHIMA ; S. HATA ; T. YASUDA.** Blood plasma separation and extraction from a minute amount of blood using dielectrophoretic and capillary forces. *Sens. Actuators B,* 2010, vol. 145, 561-569